# EUROPEAN PATENT SPECIFICATION

(11) **EP 4 164 699 B1**
(45) Date of publication and mention of the grant of the patent: **11.09.2024**
(21) Application number: 21732005.0
(22) Date of filing: 09.06.2021
(51) Int. Cl.: A61L 2/22, A61L 2/24, F24F 11/00, F24F 11/30, B05B 12/12

(54) **DISINFECTION DEVICE**
DESINFEKTIONSVORRICHTUNG
DISPOSITIF DE DÉSINFECTION

(30) Priority: 11.06.2020 DK PA202000682; 28.04.2021 DK PA202100425
(43) Date of publication of application: 19.04.2023
(73) Proprietor: Autonomous Units Aps, 5550 Langeskov (DK)
(72) Inventor: KJÆR THINGGAARD, Flemming, 5550 Langeskov (DK)
(74) Representative: Andreasen, Søren Laursen Vasegaard
(86) International application number: PCT/EP2021/065417
(87) International publication number: WO 2021/250073

(56) References cited:
- EP-A1- 3 412 317
- WO-A2-2019/075176
- CN-A- 111 001 025
- CN-A- 113 142 169

## Description

### Field of invention

The present invention relates to a disinfection device and a method for disinfecting one or more rooms of a building.

### Prior art

US20160271803A1 discloses a robot platform for remotely controlled and autonomous disinfection of a facility. The robot platform comprises a mobile unit configured to move the robot platform. The robot platform also comprises a disinfection module having a plurality of ultraviolet radiation emitters disposed above the drive mechanism. The robot platform, however, is not capable of sufficiently effectively disinfect building rooms because the ultraviolet radiation will not reach all surfaces.

US2003030398A1 discloses a robot system for performing a function in an area, the area having an area layout including at least one area segment. In one embodiment, the robot system comprising disinfectant applicator device configured to disinfect the area. The robot system, however, is not capable of disinfecting the area autonomously because not all area specific parameters (including room dimensions) are not detected by the robot system.

WO2019075176A2 discloses a method for disinfecting surfaces within a volumetric space using a peracid. The peracid is formed in a reaction layer in situ on the surface by sequentially dispersing a first composition comprising a peroxide compound and a first composition comprising an organic acid compound onto the surface, thereby preventing the peracid from being formed until the peroxide and organic acid contact each other on the surface. Delivery systems are provided for sequentially applying liquid compositions in a time-dependent manner, including associated software and hardware. An Internet of Things and single board computer assemblies can be utilized to control the sequential application of two or more liquid compositions in a time-dependent manner. This solution, however, is not suited for taking into account the volume of the space in a sufficiently manner. Accordingly, it would be desirable to have an alternative solution.

Further examples are disclosed by CN111001025 and EP18176052.

Thus, there is a need for a disinfection device and a method for disinfecting one or more rooms of a building which reduces or even eliminates the above-mentioned disadvantages of the prior art.

It is an object of the invention to provide a disinfection device that is capable of sufficiently effective disinfect building rooms in an autonomously manner.

It is an object of the invention to provide a method for disinfecting one or more rooms of a building, wherein the method sufficiently effective disinfects building rooms in an autonomously manner.

### Summary of the invention

The object of the present invention can be achieved by a disinfection device as defined in claim 1, by a system as defined in claim 7 and by a method as defined in claim 9. Preferred embodiments are defined in the dependent subclaims, explained in the following description and illustrated in the accompanying drawings.

The disinfection unit according to the invention is a disinfection unit configured to be operated in a room having a ceiling, wherein the disinfection unit comprises one or more nozzles each being arranged to generate a jet and hereby spray a disinfection fluid into the room, wherein the disinfection unit comprises a control unit configured to calculate a quantity of disinfection fluid to be sprayed into the room on the basis of a predefined required degree of purity, wherein the disinfection unit:
a) comprises a mobile device or
b) is configured to be electrically and mechanically connected to a mobile device,

wherein the mobile device is configured to move the disinfection unit, wherein the disinfection unit comprises a position determination device configured to determine the position of the disinfection unit, wherein the disinfection unit comprises a detection unit arranged and configured to detect a height of the room,
wherein the disinfection unit is configured to carry out a height detection while the mobile device is moved along a predefined path, wherein the predefined path is selected in such a manner that at least one height measurement is available for each square meter of the ceiling.

Hereby, it is possible to provide a disinfection device that is capable of sufficiently effective disinfect building rooms in an autonomously manner. While the prior art solutions require manually settings (e.g. that manual measurements are made and entered) before the disinfection device is operated.

The disinfection unit is configured to be operated in a room having a ceiling.

The one or more nozzles each arranged to generate a jet and hereby spray a disinfection fluid into the room. The pressure for creating the jet may be generated by a pump or compressor.

In one embodiment, the speed of the pump or compressor is regulated by a regulator. In one embodiment, the regulator is a frequency converter. In one embodiment, the regulator is a

Pulse width modulation (PWM) controller. The PWM controller is configured to reducing the average power delivered by an electrical signal, by chopping it up into discrete parts. Hereby, the duty cycle can be changed.

The control unit is configured to calculate a quantity of disinfection fluid to be sprayed into the room on the basis of a predefined required degree of purity and the volume of the room.

The control unit is configured to calculate the estimates time for spraying the disinfection fluid into the room on the basis of a predefined required degree of purity and the volume of the room.

In one embodiment, the disinfection unit comprises a mobile device. This means than the disinfection unit has an integrated mobile device that is configured to move the disinfection unit.

In one embodiment, the disinfection unit is configured to be electrically and mechanically connected to a mobile device. In this embodiment, the mobile device is not integrated into the disinfection unit.

The disinfection unit comprises a position determination device configured to determine the position of the disinfection unit. In one embodiment, the position determination device is integrated in the disinfection unit.

In one embodiment, the position determination device is integrated in the mobile device. Hereby, the disinfection unit can benefit from using the determination device is integrated in the mobile device.

The disinfection unit comprises a detection unit arranged and configured to detect at least one height of the room.

In a preferred embodiment, the disinfection unit comprises a detection unit arranged and configured to detect all heights of the room.

The height detection may be done by the disinfection unit while the mobile device moves the disinfection unit within the room.

According to the invention, the disinfection unit is configured to carry out a height detection while the mobile device is moved along a predefined path. The predefined path should be selected in such a manner that a sufficiently large number of height measurements are available in order to ensure that at predefined number of height measurements are available for square meter of the ceiling. According to the invention, the predefined number of height measurements available is at least 1 for each square meter of the ceiling. In one embodiment, the predefined number of height measurements available is at least 2 for each square meter of the ceiling.

In one embodiment, the disinfection unit comprises a housing provided with a plurality of nozzles, wherein the nozzles are directed in different directions. In one embodiment, the directions of the corresponding jets are directed in different directions.

Hereby, it is possible to provide a distribution of the disinfection fluid.

In one embodiment, the disinfection unit is configured to carry out a position specific distribution of the disinfection fluid so that the disinfection fluid is distributed in dependency of position specific height measurements of the detection unit. Hereby, it is possible to take different height distributions of a room into account.

In one embodiment, the disinfection unit is configured to carry out a position specific distribution of the disinfection fluid in such a manner that the volume of the disinfection fluid is distributed in dependency of one or more height measurements carried out by the detection unit while the disinfection unit is within 2 meters from an actual position.

In one embodiment, the disinfection unit is configured to carry out a position specific distribution of the disinfection fluid in such a manner that the volume of the disinfection fluid is distributed in dependency of one or more height measurements carried out by the detection unit while the disinfection unit is within 1 meter from the actual position.

By the term "the actual position" is meant the horizontal coordinates of the disinfection unit in the building. The "the actual position" would typically be defined by a X value and a Y value, wherein X and Y represents two orthogonal horizontal axes extending along the floor of the room.

In one embodiment, the disinfection unit is configured to distribute the distribute the disinfection fluid evenly in the space independently of the height.

In one embodiment, the nozzles are symmetrically arranged on such a manner that the jet of a central nozzle extends along the frontal plane of the disinfection unit.

In one embodiment, the jet of nozzles adjacent to the central nozzle are angled relative to the frontal plane of the disinfection unit.

I one embodiment, the angle α between the first adjacent nozzle and the jet of the central nozzle corresponds to the angle β between the second adjacent nozzle and the jet of the central nozzle.

In one embodiment, the control unit is arranged and configured to receive signals from one or more external devices, wherein the control unit is configured to control the activity of the disinfection unit on the basis of these signals.

Hereby, it is possible to take external conditions into consideration when operating the disinfection unit.

In one embodiment, the signals are wirelessly sent signals.

In one embodiment, the control unit is arranged and configured to receive signals from one or more external devices, wherein the control unit is configured to control the activity of the mobile device on the basis of these signals.

In one embodiment, the one or more external devices includes a communication unit that receives signals from the ventilation system of the building, in which the disinfection unit is operated.

In one embodiment, the one or more external devices includes a reader box arranged to detect when a member of staff leaves or enters the building, wherein the reader box comprises a key reader configured to read a key member used by a person to acknowledge that the person enters the building or leaves the building.

In one embodiment, the control unit is configured to stop the disinfection unit if the control unit receives a signal that indicates that a person is in the room to be disinfected.

In one embodiment, the control unit is configured to stop the disinfection unit if the control unit receives a signal that indicates that the ventilation system is turned on.

In one embodiment, the control unit is configured to stop the disinfection unit if the control unit receives a signal that a door is being opened. The signal may be transmitted by a sensor arranged to detect opening of the door.

In one embodiment, the control unit is configured to stop the disinfection unit if the control unit receives a signal that a person enters a room in the building, in which the disinfection is arranged. The signal may be transmitted by a motion arranged to detect nearby motion. In one embodiment, the motion sensor is a passive infrared sensor (PIR sensor) that is configured to measure infrared (IR) light radiating from objects (such a person) in its field of view.

The disinfection system according to the invention is a disinfection system that comprises:
- a disinfection unit according to the invention;
- a ventilation system and
- one or more communication units arranged and configured to detect the activity of the ventilation system,
wherein the disinfection system is configured to control the activity of the disinfection unit on the basis of the detected activity of the ventilation system.

Hereby, the disinfection system is capable of sufficiently effective disinfect building rooms in an autonomously manner and take the activity of the ventilation system into consideration.

The one or more communication units is integrated in the ventilation system.

In one embodiment, the disinfection system comprises a reader box arranged and configured to read a key member used by persons entering or leaving the building in which the room is placed, wherein the communication unit is arranged and configured to detect the activity of a reader box, wherein the disinfection system is configured to control the activity of the disinfection unit on the basis of the detected activity of the reader box.

Hereby, it is possible to ensure that the disinfection unit is only operated when no persons are present in the room or in the building, in which the room is located.

By the term "activity of a reader box" is meant events detected by the activity of a reader box. Such events may include that a person acknowledges that he or she is leaving the building or is entering the building.

In one embodiment, the communication unit is integrated in the reader box. Accordingly, the reader box sends signals directly or indirectly (via an intermedia device) to the disinfection unit.

In one embodiment, the disinfection system is configured to stop the disinfection unit if it is detected that one or more of the ventilation devices are turned on. This can be done by applying a control algorithm that only allows the disinfection unit to be operated if it has been verified that no "turned on" signals are received form any of the ventilation devices.

In one embodiment, the disinfection system is configured to stop the disinfection unit if it is detected that a door is being opened. The signal may be transmitted by a sensor arranged to detect opening of the door.

In one embodiment, the disinfection system is configured to stop the disinfection unit if it is detected that a person enters a room in the building, in which the disinfection is arranged. The signal may be transmitted by a motion arranged to detect nearby motion. In one embodiment, the motion sensor is a passive infrared sensor (PIR sensor) that is configured to measure infrared (IR) light radiating from objects (such a person) in its field of view.

The method according to the invention is a method for disinfecting a room having a ceiling, wherein the method uses a disinfection unit that comprises one or more nozzles each being arranged to generate a jet and hereby spray a disinfection fluid into the room, wherein the disinfection unit comprises a control unit configured to calculate a quantity of disinfection fluid to be sprayed into the room on the basis of a predefined required degree of purity, wherein the disinfection unit:
a) comprises a mobile device or
b) is configured to be electrically and mechanically connected to a mobile device,

wherein the mobile device is configured to move the disinfection unit, wherein the disinfection unit comprises a position determination device configured to determine the position of the disinfection unit, wherein the method comprises the step of detecting a height of the room by means of a detection unit of the disinfection unit,
wherein the disinfection unit is configured to carry out a height detection while the mobile device is moved along a predefined path, wherein the predefined path is selected in such a manner that at least one height measurement is available for each square meter of the ceiling.

Hereby, it is possible to provide a method that sufficiently effective disinfects building rooms in an autonomously manner.

It may be an advantage that the method comprises the step of controlling the activity of the disinfection unit on the basis of signals received from one or more external devices.

In one embodiment, the step of controlling the activity of the disinfection unit is carried out on the basis of signals from the key system when the last person leaves the building, in which the room is placed.

In one embodiment, the step of controlling the activity of the disinfection unit is carried out on the basis of signals indicating the activity of a ventilation system. Hereby, it is possible to only operate the disinfection unit when the ventilation system is turned off.

In one embodiment, the step of controlling the activity of the disinfection unit is carried out on the basis of one or more signals indicating that a door is being opened. The signal may be transmitted by a sensor arranged to detect opening of the door.

In one embodiment, the step of controlling the activity of the disinfection unit is carried out on the basis of one or more signals indicating that a person enters a room in the building, in which the disinfection is arranged. The signal may be transmitted by a motion arranged to detect nearby motion. In one embodiment, the motion sensor is a passive infrared sensor (PIR sensor) that is configured to measure infrared (IR) light radiating from objects (such a person) in its field of view.

### Description of the Drawings

The invention will become more fully understood from the detailed description given herein below. The accompanying drawings are given by way of illustration only, and thus, they are not limitative of the present invention. In the accompanying drawings:
- Fig. 1A: shows a side view of a disinfection unit according to the invention;
- Fig. 1B: shows a front view of the disinfection unit shown in Fig. 1A;
- Fig. 2A: shows a perspective top view of the disinfection unit shown in Fig. 1A and in Fig. 1B;
- Fig. 2B: shows a top view of the disinfection unit shown in Fig. 1A and in Fig. 1B;
- Fig. 3A: shows a disinfection unit being operated in a first location;
- Fig. 3B: shows a disinfection unit being operated in a second location;
- Fig. 4: shows a schematic view of a disinfection system according to the invention;
- Fig. 5A: shows two-dimensional sensor data detected by sensors of a disinfection unit according to the invention in a first room;
- Fig. 5B: shows two-dimensional sensor data detected by sensors of a disinfection unit according to the invention in a second room;
- Fig. 6: shows a disinfection unit according to the invention operated in a room;
- Fig. 7A: shows a top view of a disinfection unit according to the invention operated in a room and
- Fig. 7B: shows a side view of a disinfection unit according to the invention operated in a room.

### Detailed description of the invention

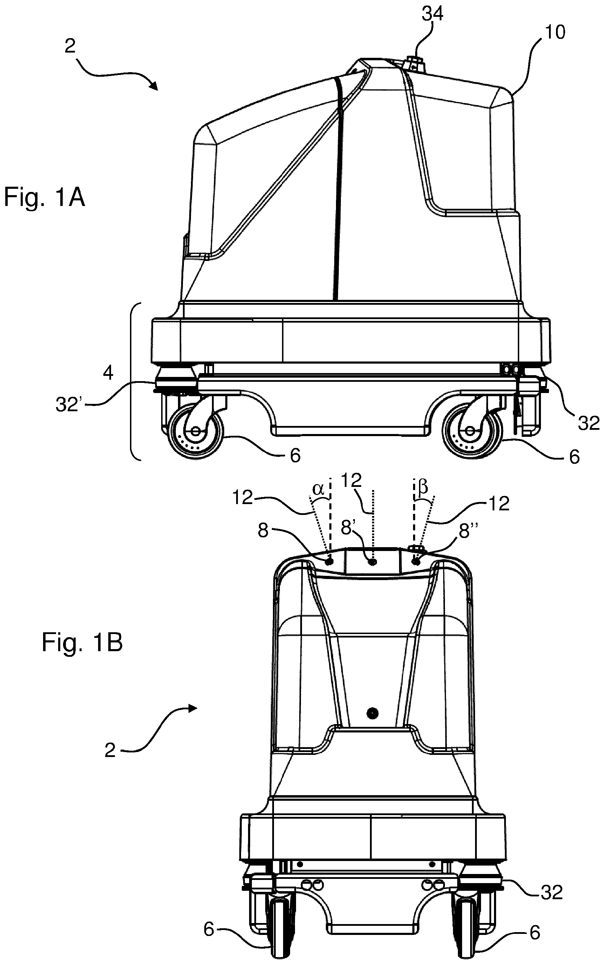

Referring now in detail to the drawings for the purpose of illustrating preferred embodiments of the present invention, a disinfection unit 2 of the present invention is illustrated in Fig. 1A.

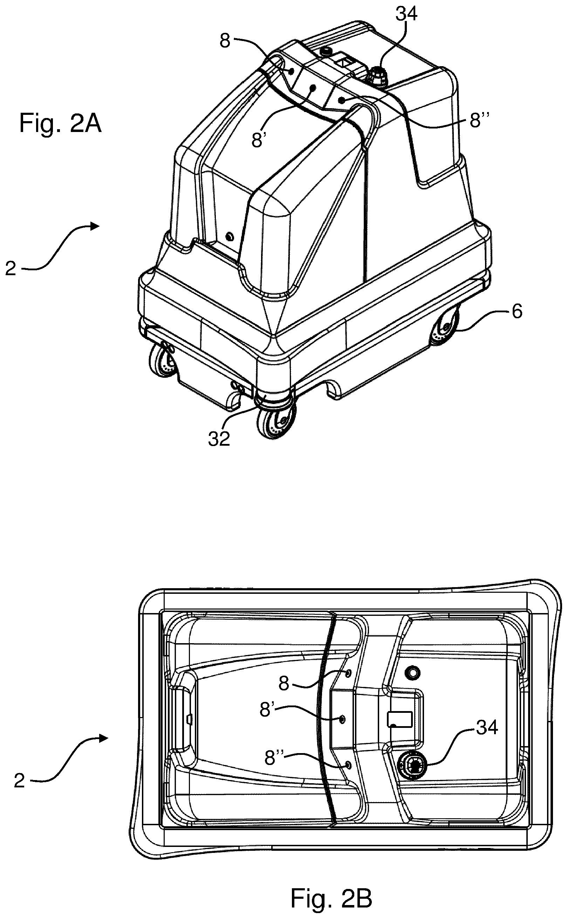

Fig. 1A illustrates a schematic side view of a disinfection unit 2 according to the invention. Fig. 1B illustrates a front view of the disinfection unit shown in Fig. 1A. Fig. 2A illustrates a perspective top view of the disinfection unit shown in Fig. 1A and in Fig. 1B. Fig. 2B illustrates a top view of the disinfection unit shown in Fig. 1A and in Fig. 1B.

The disinfection unit 2 comprises a housing 10 provided with a compartment for a disinfection fluid. A detection unit 34 is arranged at the upper portion of the hosing 10. The detection unit 34 is arranged and configured to detect the height H₁, H₂, H₃ of the room, in which the disinfection unit 2 operated. In one embodiment, the detection unit 34 is a laser distance sensor. A laser distance sensor ensures high process stability. In one embodiment, the detection unit 34 is an ultrasonic distance sensor.

The disinfection unit 2 comprises three nozzles 8, 8', 8" arranged at the upper portion of the housing 10. Each nozzle 8, 8', 8" is arranged to generate a jet 12 and hereby spray a disinfection fluid into the room in which the disinfection unit 2 is operated.

In Fig. 1B it can be seen that the central nozzles 8, 8', 8" are symmetrically arranged. The jet of the central nozzle 8' extends along the frontal plane of the disinfection unit 2. The jet 12 of nozzles 8, 8" adjacent to the central nozzle 8' are angled relative to the frontal plane of the disinfection unit 2. It can be seen that the angle a between the first nozzle 8 and the jet 12 of the central nozzle 8' corresponds to the angle β between the third nozzle 8" and the jet 12 of the central nozzle 8'.

The disinfection unit 2 is arranged on a mobile device 4 that comprises a plurality of ground engaging wheels 6. The mobile device 4 comprises sensors 32, 32' configured to detect any objects present in the room, in which the disinfection unit 2 is operated. In one embodiment, the sensors 32, 32' are laser sensors.

The disinfection unit 2 comprises a control unit that is arranged and configured to receive and process data. The disinfection unit 2 is configured to be mechanically and electrically connected to the mobile device 4. Hereby, it is possible to send sensor data detected by the mobile device 4 to the control unit. Accordingly, the disinfection unit 2 can process these sensor data and use them to calculate the volume of the room, in which the disinfection unit 2 is operated.

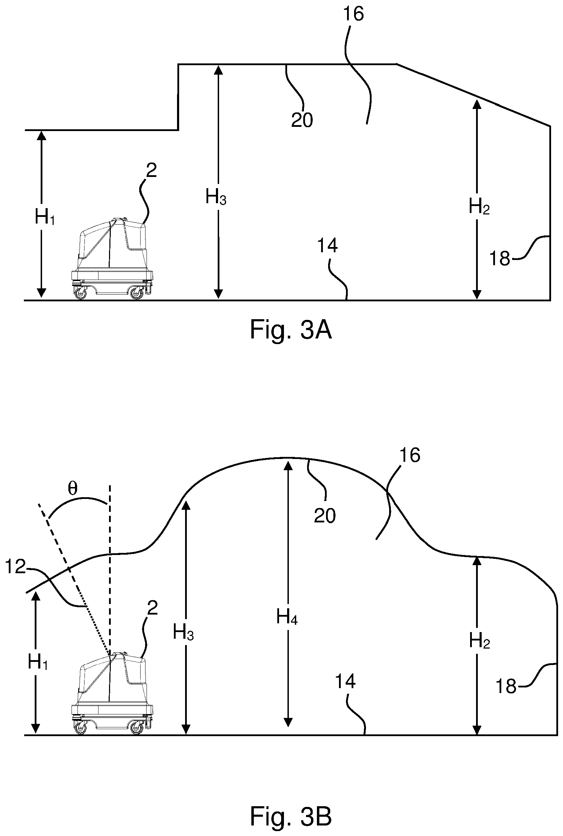

Fig. 3A illustrates a disinfection unit 2 according to the invention being operated in a room 16. The room 16 comprises a floor 14, a wall 18 and a ceiling 20. The ceiling 20 comprises several different sections of different heights H₁, H₂, H₃.

The disinfection unit 2 is arrange din a first section of the room 16 having a first constant height is H₁. This section is located next to a section having a larger constant height is H₃. This section is located next to a section having a decreasing height is H₂. Decreasing from H₃ to H₁.

The detection unit 2 comprises a detection unit that is arranged and configured to detect the heights H₁, H₂, H₃ of the room 16. Accordingly, the disinfection unit 2 is configured to detect determine the dimension of the room 16 so that the volume of the room 16 can be automatically determined.

Fig. 3B illustrates a disinfection unit 2 being operated in a second room 16 defined by a floor 14, a wall 18 as well as a ceiling 20 having a central arch arranged between side portions. It can be seen that the height varies. In the side portions the height H₁, H₂ is lower than the heights H₃, H₄ in the central arch.

The disinfection unit 2 comprises nozzles arranged and configured to spray jets 12 in an upwards direction. The angle θ between the spray directing and vertical is indicated. In one embodiment, the angle θ between the spray directing and vertical is 5-45 degrees.

In one embodiment, the angle θ between the spray directing and vertical is in the range 10-20 degrees.

In one embodiment, the angle θ between the spray directing and vertical is in the range 20-30 degrees.

In one embodiment, the angle θ between the spray directing and vertical is in the range 30-40 degrees.

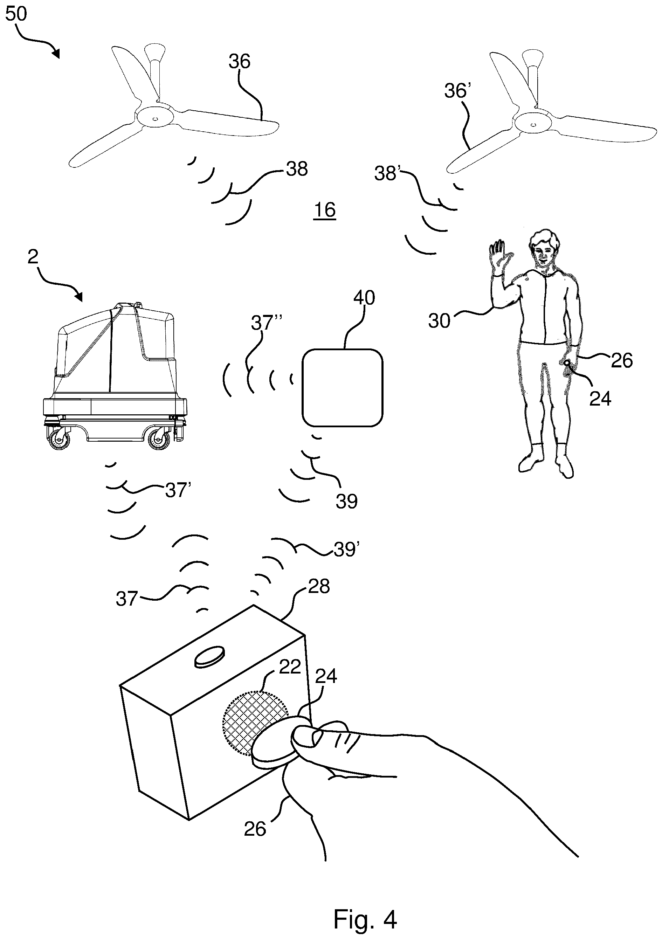

Fig. 4 illustrates a schematic view of a disinfection system 50 according to the invention. The disinfection system 50 comprises a disinfection unit 2 corresponding to the one shown in and explained with reference to the preceding figures.

The disinfection unit 2 is configured to carry out the most efficient disinfection and in the same time avoid that personnel are exposed for the disinfection fluid sprayed by the disinfection unit 2. This is achieved by detecting the activity of ventilation devices 36, 36' installed in the room 16 to be disinfected by the disinfection unit 2. It should be noted that even though the ventilation devices 36, 36' are illustrated as ceiling mounted fans, the ventilation devices 36, 36' may be any type of ventilation device including ventilating devices arranged and configured to ventilate the room 16. Accordingly, the ventilation devices 36, 36' may be arranged and configured to replace air from the room 16 with air from outside.

The disinfection system 50 comprises a communication unit 40 arranged and configured to detect the activity of ventilation devices 36, 36'. This is done by receiving signals 38, 38' sent from the ventilation devices 36, 36'.

The communication unit 40 is also arranged and configured to detect the activity of a reader box 28. The reader box 28 is used by the staff 30 working in the building in which room 16 is placed. When a member of staff leaves or enters the building, a key member 24 is used to acknowledge that the person 30 enters the building or leaves the building. The reader box 28 comprises a key reader 22 integrated in or attached to the reader box 28. The reader box 28 is arranged and configured to send signals 37 to the disinfection unit 2 (preferably to a control unit of the). In one embodiment, the reader box 28 is arranged and configured to send signals 37 to the communication unit 40, wherein the communication unit 40 is arranged and configured to transmit received signals 38', 39 to the disinfection unit 2 (preferably to a control unit of the).

In practice, the person 30 holds a key member 24 in his hand 26 and holds the key member 24 in close proximity to the key reader 22 of the reader box 28 in order to acknowledge entrance into and/or exit from the building, in which the room 16 is placed.

In one embodiment, the disinfection system 50 is configured to stop the disinfection unit 2 if it is detected that one or more of the ventilation devices 36, 36' are turned on. This can be done by applying a control algorithm that only allows the disinfection unit 2 to be operated if it has been verified that no "turned on" signals are received form any of the ventilation devices 36, 36'.

In one embodiment, the disinfection system 50 is configured to stop the disinfection unit 2 if it is detected that one or more persons 30 are present in one or more predefined rooms 16 of the building. This can be done by applying a control algorithm that only allows the disinfection unit 2 to be operated if it has been verified that no persons 30 are present in one or more predefined rooms 16 of the building.

In one embodiment, the disinfection system 50 is configured to stop the disinfection unit 2 if it is detected that one or more persons 30 are present in the building. This can be done by applying a control algorithm that only allows the disinfection unit 2 to be operated if it has been verified that no persons 30 are present building.

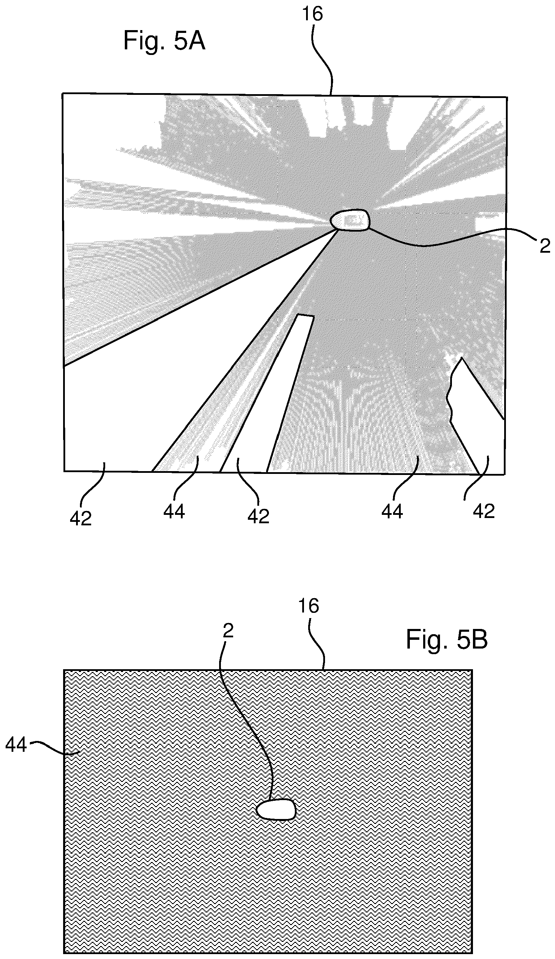

Fig. 5A illustrates two-dimensional sensor data of a room 16 detected by sensors of a disinfection unit according to the invention. The two-dimensional sensor data may be detected by sensors 32, 32' as shown in and explained with reference to Fig. 1A, Fig. 1B and Fig. 2A.

It can be seen that the disinfection unit 2 is arranged close to the central part of the room 16. Moreover, it can be seen that the two-dimensional sensor data comprises non-visible areas 42 as well as visible areas 44. The non-visible areas 42 detected by the disinfection unit 2 may be caused by an object that provides shade.

Fig. 5B illustrates two-dimensional sensor data of another room 16 detected by sensors of a disinfection unit according to the invention. The two-dimensional sensor data may be detected by sensors 32, 32' as shown in and explained with reference to Fig. 1A, Fig. 1B and Fig. 2A.

The disinfection unit 2 is arranged centrally in the room 16. Two-dimensional sensor data comprises no non-visible areas but only visible areas 44. This indicates that no objects are placed on the floor of the room 16.

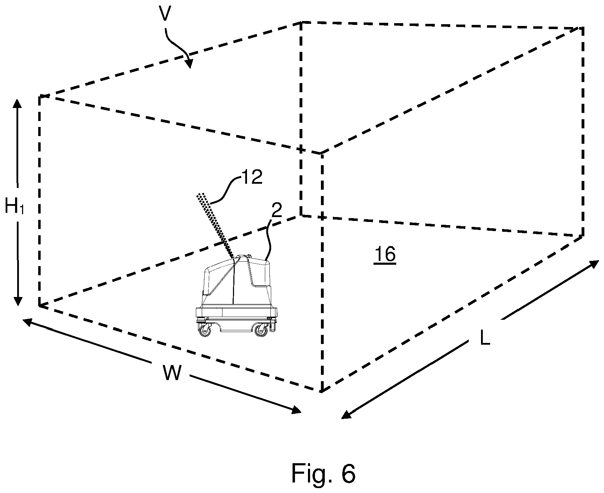

Fig. 6 illustrates a disinfection unit 2 according to the invention operated in a room 16. The room 16 has rectangular floor having a length L and a width W. Accordingly, the area of the floor can be calculated as the product between the length L and the width W. The room 16 has a height H₁. Therefore, the disinfection unit 2 can automatically detect that the volume V of the room 16 is given by the product by the length L, the width W and the height H₁.

In practice, the disinfection unit 2 can initially automatically detect the dimensions of the room 16. Accordingly, the volume of the room can be calculated on the basis of the detected dimensions of the room 16.

When the volume of the room has been calculated, the disinfection unit 2 can automatically disinfect the room 16 in an efficient manner.

The disinfection unit 2 can by means of the mobile device carrying it move freely in the room 16. The control unit (not shown) of the disinfection unit 2 is configured to calculate the volume V of the room 16 on the basis of the data collected by means of the sensors and detection unit of the disinfection unit 2.

The control unit of the disinfection unit 2 is configured to calculate the disinfection time required by the disinfection unit 2 to obtain the desired degree of purity.

Accordingly, the disinfection unit 2 is configured to carry out the disinfection process in the shortest possible time and with the greatest possible precision. The process can be carried out automatically by the disinfection unit 2 without requiring any staff.

In a preferred embodiment, the disinfection unit 2 is configured to select the pressure applied to create the jet 12 on the basis of measurements carried out by any of the sensors and detection unit of the disinfection unit 2.

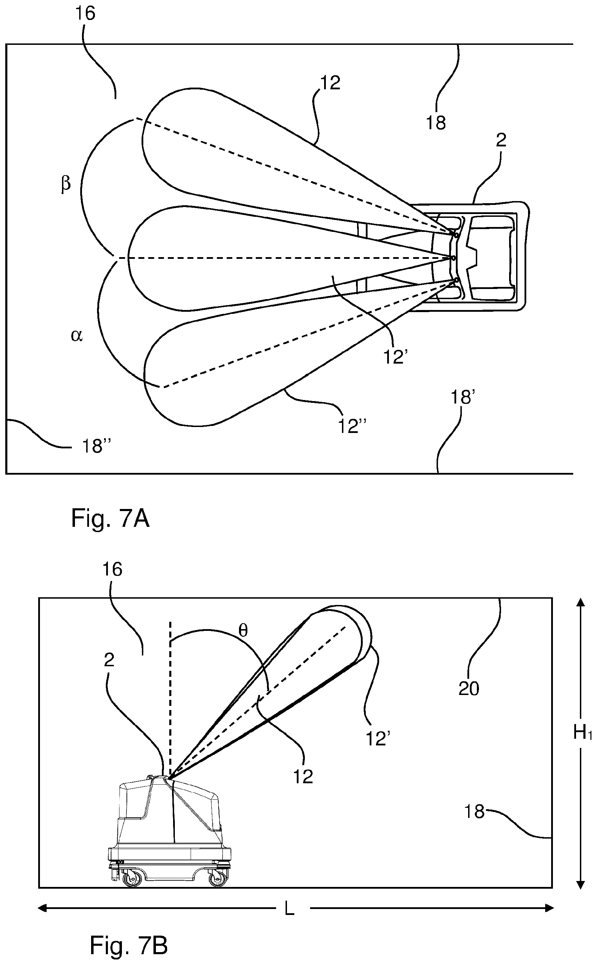

Fig. 7A illustrates a top view of a disinfection unit 2 according to the invention operated in a room 16. The room 16 has several walls 18, 18', 18" defining the boundaries of the room 16. It can be seen that the disinfection unit 2 comprises three nozzles each generating a jet 12, 12', 12". The direction of the jets 12, 12', 12" differ. The angle (viewed in the horizontal plane) α, β between adjacent jets 12, 12', 12" are approximately 20 degrees.

The disinfection unit 2 configured to be maintained in a zone that ensures that the jets 12, 12', 12" do not spray directly on any of the walls 18, 18', 18". Hereby, it is possible to prevent the disinfection fluid from condensing on any wall surface.

Fig. 7B illustrates a side view of a disinfection unit 2 according to the invention operated in a room 16 having a wall 18 and a ceiling 20. The disinfection unit 2 comprises several nozzles each generating a jet 12, 12'. The angle θ of the jets 12, 12' (viewed in the vertical plane) relative to vertical is approximately 70 degrees.

The disinfection unit 2 configured to be maintained in a zone that ensures that the jets 12, 12' do not spray directly on the ceiling 20. Hereby, it is possible to prevent the disinfection fluid from condensing on the ceiling 20.

The height H₁ of the ceiling is indicated. If the disinfection unit 2 is moved into a room, in which the height of the ceiling is different, the detection unit of the disinfection unit 2 will detect the height and adjust the pressure of the nozzles. Hereby, the disinfection unit 2 is capable of controlling the jets 12, 12' in such a manner that no disinfection fluid will condense on the ceiling 20. The disinfection unit 2 comprises one or more other devices (e.g. sensors) capable of detecting the distance between the disinfection unit 2 and any adjacent wall 18. Accordingly, the disinfection unit 2 is capable of controlling the jets 12, 12' in such a manner that no disinfection fluid will condense on any wall 18.

### List of reference numerals

- 2: Disinfection unit
- 4: Mobile device
- 6: Wheel
- 8, 8', 8": Nozzle
- 10: Housing
- 12, 12', 12": Jet
- 14: Floor
- 16: Room
- 18, 18', 18": Wall
- 20: Ceiling
- 22: Key reader
- 24: Key member
- 26: Hand
- 28: Reader box
- 30: Person
- 32, 32': Sensor
- 34: Detection unit
- 36: Ventilation unit
- 37, 37', 37": Signal
- 38, 38': Signal
- 39, 39': Signal
- 40: Communication unit
- 42: Non-visible area
- 44: Visible area
- 50: Disinfection system
- H₁, H₂: Height
- H₃, H₄: Height
- α, β, θ: Angle
- W: Width
- L: Length
- V: Volume

## Claims

1. A disinfection unit (2) configured to be operated in a room (16) having a ceiling (20), wherein the disinfection unit (2) comprises one or more nozzles (8, 8', 8") each being arranged to generate a jet (12) and hereby spray a disinfection fluid into the room (16), wherein the disinfection unit (2) comprises a control unit configured to calculate a quantity of disinfection fluid to be sprayed into the room (16) on the basis of a predefined required degree of purity, wherein the disinfection unit (2):
a) comprises a mobile device (4) or
b) is configured to be electrically and mechanically connected to a mobile device (4),
wherein the mobile device (4) is configured to move the disinfection unit (2), wherein the disinfection unit (2) comprises a position determination device configured to determine the position of the disinfection unit (2),
wherein the disinfection unit (2) comprises a detection unit (34) arranged and configured to detect a height (H₁, H₂, H₃) of the room (16),
**characterised in that** the disinfection unit (2) is configured to carry out a height detection while the mobile device (4) is moved along a predefined path, wherein the predefined path is selected in such a manner that at least one height measurement is available for each square meter of the ceiling (20).

2. A disinfection unit (2) according to claim 1, **characterised in that** the disinfection unit (2) comprises a housing (10) provided with a plurality of nozzles (8, 8', 8"), wherein the nozzles (8, 8', 8") and the directions of the corresponding jets (12) are directed in different directions.

3. A disinfection unit (2) according to claim 2, **characterised in that** the disinfection unit (2) is configured to carry out a position specific distribution of the disinfection fluid so that the disinfection fluid is distributed in dependency of position specific height measurements of the detection unit (34).

4. A disinfection unit (2) according to claim 2, **characterised in that** the disinfection unit (2) is configured to carry out a position specific distribution of the disinfection fluid in such a manner that the volume of the disinfection fluid is distributed in dependency of one or more height measurements carried out by the detection unit (34) while the disinfection unit (2) is within 2 meters from an actual position.

5. A disinfection unit (2) according to one of the preceding claims, **characterised in that** the control unit is arranged and configured to receive signals (37, 37") from one or more external devices (36, 36', 40, 28), wherein the control unit is configured to control the activity of the disinfection unit (2) on the basis of these signals (37, 37").

6. A disinfection unit (2) according to claim 5, **characterised in that** the control unit is configured to stop the disinfection unit (2) if the control unit receives a signal (37, 37") that indicates that:
a) a person (30) is in the room (16) to be disinfected or
b) the ventilation system (36, 36') is turned on.

7. A disinfection system (50) comprising:
- a disinfection unit (2) according to claim 5 or 6;
- a ventilation system (36, 36') and
- one or more communication units (40) arranged and configured to detect the activity of the ventilation system (36, 36'),
wherein the disinfection system (50) is configured to control the activity of the disinfection unit (2) on the basis of the detected activity of the ventilation system (36, 36').

8. A disinfection system (50) according to claim 7, wherein the disinfection system (50) comprises a reader box (28) arranged and configured to read a key member (24) used by persons (30) entering or leaving the building in which the room (16) is placed, wherein the communication unit (40) is also arranged and configured to detect the activity of a reader box (28), wherein the disinfection system (50) is configured to control the activity of the disinfection unit (2) on the basis of the detected activity of the reader box (28).

9. A method for disinfecting a room (16) having a ceiling (20), wherein the method uses a disinfection unit (2) that comprises one or more nozzles (8, 8', 8") each being arranged to generate a jet (12) and hereby spray a disinfection fluid into the room (16), wherein the disinfection unit (2) comprises a control unit configured to calculate a quantity of disinfection fluid to be sprayed into the room (16) on the basis of a predefined required degree of purity, wherein the disinfection unit (2):
a) comprises a mobile device (4) or
b) is configured to be electrically and mechanically connected to a mobile device (4),
wherein the mobile device (4) is configured to move the disinfection unit (2), wherein the disinfection unit (2) comprises a position determination device configured to determine the position of the disinfection unit (2), wherein the method comprises the step of detecting a height (H₁, H₂, H₃) of the room (16) by means of a detection unit (34) of the disinfection unit (2),
**characterised in that** the disinfection unit (2) is configured to carry out a height detection while the mobile device (4) is moved along a predefined path, wherein the predefined path is selected in such a manner that at least one height measurement is available for each square meter of the ceiling (20).

10. A method according to claim 9, wherein the method comprises the step of controlling the activity of the disinfection unit (2) on the basis of signals (37, 37") received from one or more external devices (36, 36', 40, 28).

## Patentansprüche

1. Desinfektionseinheit (2), die für den Betrieb in einem Raum (16) mit einer Decke (20) konfiguriert ist, wobei die Desinfektionseinheit (2) eine oder mehrere Düsen (8, 8', 8") umfasst, die jeweils so eingerichtet sind, dass sie einen Strahl (12) erzeugen und hierdurch ein Desinfektionsfluid in den Raum (16) sprühen, wobei die Desinfektionseinheit (2) eine Steuereinheit umfasst, die konfiguriert ist, um eine Menge an in den Raum (16) zu sprühendem Desinfektionsfluid auf der Grundlage von einem vordefinierten erforderlichen Reinheitsgrad zu berechnen, wobei die Desinfektionseinheit (2):
a) eine mobile Vorrichtung (4) umfasst, oder
b) konfiguriert ist, um elektrisch und mechanisch mit einer mobilen Vorrichtung (4) verbunden zu werden,
wobei die mobile Vorrichtung (4) konfiguriert ist, um die Desinfektionseinheit (2) zu bewegen, wobei die Desinfektionseinheit (2) eine Positionsbestimmungsvorrichtung umfasst, die konfiguriert ist, um die Position der Desinfektionseinheit (2) zu bestimmen,
wobei die Desinfektionseinheit (2) eine Erkennungseinheit (34) umfasst, die eingerichtet und konfiguriert ist, um eine Höhe (H₁, H₂, H₃) des Raums (16) zu erfassen,
**dadurch gekennzeichnet, dass** die Desinfektionseinheit (2) konfiguriert ist, um eine Höhenerkennung durchzuführen, während die mobile Vorrichtung (4) entlang eines vordefinierten Wegs bewegt wird, wobei der vordefinierte Weg auf eine solche Weise ausgewählt wird, dass für jeden Quadratmeter der Decke (20) mindestens eine Höhenmessung verfügbar ist.

2. Desinfektionseinheit (2) nach Anspruch 1, **dadurch gekennzeichnet, dass** die Desinfektionseinheit (2) ein Gehäuse (10) umfasst, das mit einer Vielzahl von Düsen (8, 8', 8") versehen ist, wobei die Düsen (8, 8', 8") und die Richtungen der entsprechenden Strahlen (12) in unterschiedliche Richtungen gerichtet sind.

3. Desinfektionseinheit (2) nach Anspruch 2, **dadurch gekennzeichnet, dass** die Desinfektionseinheit (2) konfiguriert ist, um eine positionsspezifische Verteilung des Desinfektionsfluids derart durchzuführen, dass das Desinfektionsfluid in Abhängigkeit von positionsspezifischen Höhenmessungen der Erfassungseinheit (34) verteilt wird.

4. Desinfektionseinheit (2) nach Anspruch 2, **dadurch gekennzeichnet, dass** die Desinfektionseinheit (2) konfiguriert ist, um eine positionsspezifische Verteilung des Desinfektionsfluids auf eine solche Weise durchzuführen, dass das Volumen des Desinfektionsfluids in Abhängigkeit von einer oder mehreren Höhenmessungen verteilt wird, die von der Erfassungseinheit (34) durchgeführt werden, während sich die Desinfektionseinheit (2) innerhalb von 2 Metern von einer tatsächlichen Position befindet.

5. Desinfektionseinheit (2) nach einem der vorstehenden Ansprüche, **dadurch gekennzeichnet, dass** die Steuereinheit so eingerichtet und konfiguriert ist, um Signale (37, 37") von einer oder mehreren externen Vorrichtungen (36, 36', 40, 28 ) zu empfangen, wobei die Steuereinheit konfiguriert ist, um die Aktivität der Desinfektionseinheit (2) auf der Grundlage dieser Signale (37, 37") zu steuern.

6. Desinfektionseinheit (2) nach Anspruch 5, **dadurch gekennzeichnet, dass** die Steuereinheit konfiguriert ist, um die Desinfektionseinheit (2) zu stoppen, falls die Steuereinheit ein Signal (37, 37") empfängt, das anzeigt, dass:
a) sich eine Person (30) in dem zu desinfizierenden Raum (16) befindet, oder
b) das Belüftungssystem (36, 36') eingeschaltet ist.

7. Desinfektionssystem (50), umfassend:
- eine Desinfektionseinheit (2) nach Anspruch 5 oder 6;
- ein Lüftungssystem (36, 36') und
- eine oder mehrere Kommunikationseinheiten (40), die eingerichtet und konfiguriert sind, um die Aktivität des Lüftungssystems (36, 36') zu erfassen,
wobei das Desinfektionssystem (50) konfiguriert ist, um die Aktivität der Desinfektionseinheit (2) auf der Grundlage der erfassten Aktivität des Lüftungssystems (36, 36') zu steuern.

8. Desinfektionssystem (50) nach Anspruch 7, wobei das Desinfektionssystem (50) ein Lesegerät (28) umfasst, das eingerichtet und konfiguriert ist, um ein Schlüsselelement (24) zu lesen, das von Personen (30) verwendet wird, die das Gebäude betreten oder verlassen, in dem sich der Raum (16) befindet, wobei die Kommunikationseinheit (40) auch eingerichtet und konfiguriert ist, um die Aktivität eines Lesegeräts (28) zu erfassen, wobei das Desinfektionssystem (50) konfiguriert ist, um die Aktivität der Desinfektionseinheit (2) auf der Grundlage der erfassten Aktivität des Lesegeräts (28) zu steuern.

9. Verfahren zum Desinfizieren eines Raums (16) mit einer Decke (20) konfiguriert ist, wobei die Desinfektionseinheit (2) eine oder mehrere Düsen (8, 8', 8") umfasst, die jeweils so eingerichtet sind, dass sie einen Strahl (12) erzeugen und hierdurch ein Desinfektionsfluid in den Raum (16) sprühen, wobei die Desinfektionseinheit (2) eine Steuereinheit umfasst, die konfiguriert ist, um eine Menge an in den Raum (16) zu sprühendem Desinfektionsfluid auf der Grundlage von einem vordefinierten erforderlichen Reinheitsgrad zu berechnen, wobei die Desinfektionseinheit (2):
a) eine mobile Vorrichtung (4) umfasst, oder
b) konfiguriert ist, um elektrisch und mechanisch mit einer mobilen Vorrichtung (4) verbunden zu werden,
wobei die mobile Vorrichtung (4) konfiguriert ist, um die Desinfektionseinheit (2) zu bewegen, wobei die Desinfektionseinheit (2) eine Positionsbestimmungsvorrichtung umfasst, die konfiguriert ist, um die Position der Desinfektionseinheit (2) zu bestimmen, wobei das Verfahren den Schritt des Erfassens einer Höhe (H₁, H₂, H₃) des Raums (16) mittels einer Erfassungseinheit (34) der Desinfektionseinheit (2) umfasst,
**dadurch gekennzeichnet, dass** die Desinfektionseinheit (2) konfiguriert ist, um eine Höhenerkennung durchzuführen, während die mobile Vorrichtung (4) entlang eines vordefinierten Wegs bewegt wird, wobei der vordefinierte Weg auf eine solche Weise ausgewählt wird, dass für jeden Quadratmeter der Decke (20) mindestens eine Höhenmessung verfügbar ist.

10. Verfahren nach Anspruch 9, wobei das Verfahren den Schritt des Steuerns der Aktivität der Desinfektionseinheit (2) auf der Grundlage von Signalen (37, 37") umfasst, die von einer oder mehreren externen Vorrichtungen (36, 36', 40, 28) empfangen werden.

## Revendications

1. Unité de désinfection (2) configurée pour fonctionner dans une pièce (16) présentant un plafond (20), dans laquelle l'unité de désinfection (2) comprend une ou plusieurs buses (8, 8', 8"), chacune étant agencée pour générer un jet (12) et pulvériser ainsi un fluide de désinfection dans la pièce (16), dans laquelle l'unité de désinfection (2) comprend une unité de commande configurée pour calculer une quantité de fluide de désinfection à pulvériser dans la pièce (16) sur la base d'un degré de pureté requis prédéfini, dans laquelle l'unité de désinfection (2) :
a) comprend un dispositif mobile (4) ou
b) est configurée pour être connectée électriquement et mécaniquement à un dispositif mobile (4),
dans laquelle le dispositif mobile (4) est configuré pour déplacer l'unité de désinfection (2), dans laquelle l'unité de désinfection (2) comprend un dispositif de détermination de position configuré pour déterminer la position de l'unité de désinfection (2),
dans laquelle l'unité de désinfection (2) comprend une unité de détection (34) agencée et configurée pour détecter une hauteur (H₁, H₂, H₃) de la pièce (16),
**caractérisée en ce que** l'unité de désinfection (2) est configurée pour effectuer une détection de hauteur pendant que le dispositif mobile (4) est déplacé le long d'un trajet prédéfini, dans laquelle le trajet prédéfini est sélectionné de telle manière qu'au moins une mesure de hauteur soit disponible pour chaque mètre carré du plafond (20).

2. Unité de désinfection (2) selon la revendication 1, **caractérisée en ce que** l'unité de désinfection (2) comprend un corps (10) doté d'une pluralité de buses (8, 8', 8"), dans laquelle les buses (8, 8', 8") et les directions des jets correspondants (12) sont dirigées dans des directions différentes.

3. Unité de désinfection (2) selon la revendication 2, **caractérisée en ce que** l'unité de désinfection (2) est configurée pour effectuer une distribution spécifique d'une position du fluide de désinfection de sorte que le fluide de désinfection soit distribué en fonction de mesures de hauteur spécifiques d'une position de l'unité de détection (34).

4. Unité de désinfection (2) selon la revendication 2, **caractérisée en ce que** l'unité de désinfection (2) est configurée pour effectuer une distribution spécifique d'une position du fluide de désinfection de telle manière que le volume du fluide de désinfection soit distribué en fonction d'une ou plusieurs mesures de hauteur effectuées par l'unité de détection (34) en même temps que l'unité de désinfection (2) est à moins de 2 mètres d'une position réelle.

5. Unité de désinfection (2) selon l'une des revendications précédentes, **caractérisée en ce que** l'unité de commande est agencée et configurée pour recevoir des signaux (37, 37") provenant d'un ou plusieurs dispositifs externes (36, 36', 40, 28), dans laquelle l'unité de commande est configurée pour commander l'activité de l'unité de désinfection (2) sur la base de ces signaux (37, 37").

6. Unité de désinfection (2) selon la revendication 5, **caractérisée en ce que** l'unité de commande est configurée pour arrêter l'unité de désinfection (2) si l'unité de commande reçoit un signal (37, 37") qui indique que :
a) une personne (30) se trouve dans la pièce (16) à désinfecter ou
b) le système de ventilation (36, 36') est allumé.

7. Système de désinfection (50) comprenant :
- une unité de désinfection (2) selon la revendication 5 ou 6 ;
- un système de ventilation (36, 36') et
- une ou plusieurs unités de communication (40) agencées et configurées pour détecter l'activité du système de ventilation (36, 36'),
dans lequel le système de désinfection (50) est configuré pour commander l'activité de l'unité de désinfection (2) sur la base de l'activité détectée du système de ventilation (36, 36').

8. Système de désinfection (50) selon la revendication 7, dans lequel le système de désinfection (50) comprend un boîtier de lecture (28) agencé et configuré pour lire un élément clé (24) utilisé par les personnes (30) entrant ou sortant du bâtiment dans lequel se trouve la pièce (16), dans lequel l'unité de communication (40) est également agencée et configurée pour détecter l'activité d'un boîtier de lecture (28), dans lequel le système de désinfection (50) est configuré pour commander l'activité de l'unité de désinfection (2) sur la base de l'activité détectée du boîtier de lecture (28).

9. Procédé de désinfection d'une pièce (16) présentant un plafond (20), dans lequel le procédé utilise une unité de désinfection (2) qui comprend une ou plusieurs buses (8, 8', 8") chacune étant agencée pour générer un jet (12) et pulvériser ainsi un fluide de désinfection dans la pièce (16), dans lequel l'unité de désinfection (2) comprend une unité de commande configurée pour calculer une quantité de fluide de désinfection à pulvériser dans la pièce (16) sur la base d'un degré de pureté requis prédéfini, dans lequel l'unité de désinfection (2) :
a) comprend un dispositif mobile (4) ou
b) est configurée pour être connectée électriquement et mécaniquement à un appareil mobile (4),
dans lequel le dispositif mobile (4) est configuré pour déplacer l'unité de désinfection (2), dans lequel l'unité de désinfection (2) comprend un dispositif de détermination de position configuré pour déterminer la position de l'unité de désinfection (2), dans lequel le procédé comprend l'étape de détection d'une hauteur (H₁, H₂, H₃) de la pièce (16) au moyen d'une unité de détection (34) de l'unité de désinfection (2),
**caractérisé en ce que** l'unité de désinfection (2) est configurée pour effectuer une détection de hauteur pendant que le dispositif mobile (4) est déplacé le long d'un trajet prédéfini, dans lequel le trajet prédéfini est sélectionné de telle manière qu'au moins une mesure de hauteur soit disponible pour chaque mètre carré du plafond (20).

10. Procédé selon la revendication 9, dans lequel le procédé comprend l'étape de commande de l'activité de l'unité de désinfection (2) sur la base de signaux (37, 37") reçus d'un ou plusieurs dispositifs externes (36, 36', 40, 28).
